(19) Europäisches Patentamt
European Patent Office
Office européen des brevets

(11) Publication number: **0 143 307**
**B1**

(12) **EUROPEAN PATENT SPECIFICATION**

(45) Date of publication of patent specification: **04.01.89**

(51) Int. Cl.$^4$: **C 07 K 7/52,** A 61 K 37/02

(21) Application number: **84112488.6**

(22) Date of filing: **17.10.84**

(54) **Cyclic hexapeptide somatostatin analogs; process for their preparation and pharmaceutical compositions containing them.**

(30) Priority: **27.10.83 US 545982**

(43) Date of publication of application:
**05.06.85 Bulletin 85/23**

(45) Publication of the grant of the patent:
**04.01.89 Bulletin 89/01**

(84) Designated Contracting States:
**CH DE FR GB IT LI NL**

(56) References cited:
**EP-A-0 063 308**
**US-A-4 235 886**
**US-A-4 310 518**
**US-A-4 427 661**

(73) Proprietor: **MERCK & CO. INC.**
**126, East Lincoln Avenue P.O. Box 2000**
**Rahway New Jersey 07065-0900 (US)**

(72) Inventor: **Nutt, Ruth F.**
**Hill Road**
**Green Lane, PA 18054 (US)**

(74) Representative: **Blum, Rudolf Emil Ernst et al**
**c/o E. Blum & Co Patentanwälte Vorderberg 11**
**CH-8044 Zürich (CH)**

**The file contains technical information submitted after the application was filed and not included in this specification**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

Background of the Invention

Somatostatin is a tetradecapeptide incorporating a cyclic dodecapeptide, having the structure:

Ala—Gly—Cys—Lys—Asn—Phe—Phe—Trp—Lys—Thr—Phe—Thr—Ser—Cys—OH
1 2 3 4 5 6 7 8 9 10 11 12 13 14 15

and has the properties of inhibiting the release of growth hormone, inhibiting the release of insulin and glucagon and reducing gastric secretions. Somatostatin itself has a short duration of action because it is inactivated, *inter alia*, by aminopeptidases and carboxypeptidases present *in vivo*. This problem of the short duration of action has been partially solved in the prior art by preparing derivatives of somatostatin which have low solubility, thus attaining a slow release on subcutaneous injection. Once dissolved, however, the derivatives are no more stable to inactivation by aminopeptidases and carboxypeptidases than somatostatin itself.

Summary of the Invention

The present invention provides for cyclic hexapeptides which are derivatives of somatostatin in which, *inter alia*, seven of the ring amino acids are replaced by a secondary amino acid, and both of the exocyclic amino acids are removed. Further substitution and reaction of the remaining amino acids is also described. In the cyclic hexapeptides to one of the alpha carbon atoms of the six alpha amino acids which form said cyclic hexapeptide there is bonded a radical R which is a cyclohexyl radical or a phenyl radical and said radicals are either bonded directly to said alpha carbon atoms or through a $—CH_2$-bridge. When said radical comprises a cyclohexyl ring then to said ring there is directly bonded an $NH_2$ group and when said radical comprises a phenyl nucleus to said phenyl group there is bonded an $NH_2$ group through a $—CH_2$-bridge.

The cyclic hexapeptides can comprise in their structure only L-amino acids or one of the L-amino acids of said hexapeptides can be substituted through a D-amino acid, like e.g. through D-tryptophan.

Description of the Prior Art

There are already described in the prior art cyclic hexapeptides which are somatostatin analogs and in which to the alpha carbon atom of one of the amino acids of said cyclic hexapeptides there is bonded a substituent which is substituted in its ω-position with a $NH_2$ group. However, in said prior art cyclic hexapeptides said amino group is bonded to the α-carbon atom of the amino acid of the cyclic hexapeptide through an aliphatic chain and not through a group comprising a cyclohexyl ring or a phenyl nucleus. Corresponding cyclic hexapeptides are described in the following publications:

In the USA patent 4 235 886 there are described cyclic hexapeptides which are somatostatin analogs and in which said amino group is bonded to the α-carbon atom of one of the amino acids of the cyclic structure through an aliphatic chain which comprises 3—5 carbon atoms or 3 carbon atoms and a sulphur atom in any position along the chain. It can be seen from the second table in columns 11 and 12 of said publication, that the compounds described therein inhibit the gastric secretion about in the same extent as somatostatin or in a higher extent than somatostatin. It, furthermore, can be seen from the first table in column 11 of said citation, that of the totally 14 compounds, the test results of which are given in said table, only one had a far higher activity in inhibiting the release of growth hormones than somatostatin (i.e. the last compound stated in said table), while the inhibitory activity of all the other tested compounds was in the same magnitude or even far lower than the corresponding activity of somatostatin.

In the USA patent 4 310 518 there are described cyclic hexapeptides which are somatostatin analogs and in which to the α-carbon atom of one of the amino acids there is bonded an amino group through an aliphatic chain comprising 3—5 $—CH_2$-groups or an aliphatic chain comprising 3—$CH_2$-groups and a sulphur atom in any position of the chain. Two of the compounds described therein were tested as to their inhibition of the gastric secretion and one of said compounds inhibited the gastric secretion about in the same way as somatostatin, while the other showed a lower degree of inhibition (the corresponding values are stated in the second table which extends from column 11 to column 12 of said publication). It, furthermore, can be seen from the table in columns 11 and 12 that some of the tested compounds inhibit the release of growth hormones to a higher extent than somatostatin, while other compounds of said citation inhibit the release of growth hormones about as potent as somatostatin or to a lower extent.

In the European patent publication 0 063 308 there are described cyclic hexapeptides, which are somatostatin analogs and which have in their cyclic hexapeptide structure one amino acid which is a D-amino acid and not a L-amino acid. In said compounds the further amino substituent is bonded to the alpha carbon atom of an amino acid of the cyclic hexapeptide through an aliphatic chain which comprises either 3—5 $—CH_2$-groups or 3 $—CH_2$-groups and one sulphur atom in any position along the chain. The table on page 26 of said publication shows that none of the tested compounds is a far more potent inhibitor of the release of growth hormones than somatostatin.

In the USA Patent 4 427 661, which was published on January 24, 1984, there are well described cyclic hexapeptides which are somatostatin analogs and in which the further amino substituent is bonded to the carbon atom of one of the amino acids of the cyclic structure through an aliphatic chain comprising 4 —$CH_2$-groups in which chain either the second —$CH_2$-group or the third —$CH_2$-group has to be a corresponding monofluoro substituted group. Two of the compounds described therein were tested as their inhibition of the release of growth hormones, respectively the inhibition of the release of insulin and the inhibition of the release of glucagon. The inhibitory activity of said compounds was about the twofold to threefold of the corresponding activity of somatostatin.

It was the aim of the present invention to provide new somatostatin analogs which have a selective effect on the various pancreatic functions and which, accordingly, do not affect all of the insulin, growth hormone and glucagon secretions equally, but selectively affect such functions to produce a desired effect without side effect.

It was surprisingly found out that the intended aims can be achieved with a new class of cyclic hexapeptides which are somatostatin analogs and which differ structurally from all prior art somatostatin analogs having an amino substituent in that said amino substituent is bonded to the alpha carbon atom of one of the amino acids of the cyclic hexapeptides through a substituent which comprises a cyclohexyl radical or a phenyl radical, instead of the aliphatic chain of the corresponding prior art compounds.

Description of the Invention

One object of the present invention are cyclic hexapeptides having the following structural formulae

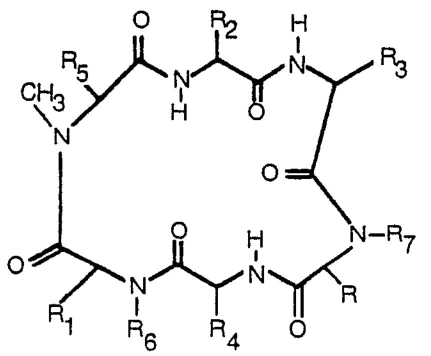

or

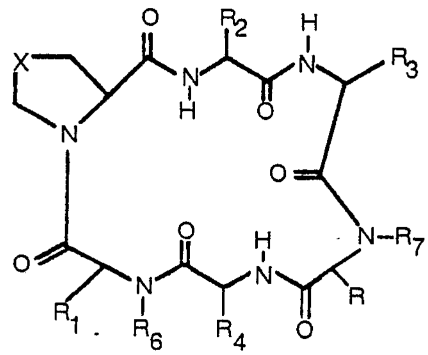

I , II

wherein

R is

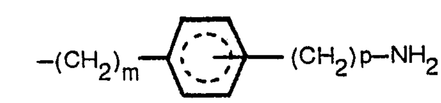

such that the broken line indicates that the ring may be either cyclohexyl or phenyl;

m is 0 or 1; and p is 0 when the ring is cyclohexyl and 1 when the ring is phenyl;

X is $(CH_2)_n$ wherein n is 0, 1 or 2, or sulfur;

$R_1$ and $R_2$ are independently lower alkyl, benzyl, substituted benzyl wherein the substituent may be one or two of loweralkyl, halogen, hydroxy, amino, nitro or loweralkoxy; and loweralkyl substituted with hydroxy, 3-indolylmethyl, carboxy, amino, guanidino, or a 5- or 6-membered heterocyclic ring;

$R_3$ is 3-indolylmethyl or substituted 3-indolylmethyl wherein the substituent may be loweralkyl, loweralkoxy or halogen;

$R_4$ is loweralkyl, hydroxyloweralkyl, benzyl, carboxyloweralkyl, aminoloweralkyl, 3-indolylmethyl or substituted benzyl or hydroxy-benzyl wherein the substituent may be loweralkyl, loweralkoxy, hydroxy, halogen, amino or nitro, or a 5- or 6-membered heterocyclic ring;

$R_5$ is loweralkyl, benzyl, or substituted benzyl wherein the substituent is loweralkyl, loweralkoxy, hydroxy, halogen, amino or nitro; and

$R_6$ and $R_7$ are independently hydrogen or methyl, and salts of the compounds of formula I or II.

The cyclic hexapeptides of formulae I and II and the salts thereof inhibit the release of glucagon, growth hormones and insulin and some of the compounds, furthermore, also inhibit the release of gastric acid secretion. Among said compounds there are to be found also such compounds which inhibit the release of growth hormones without affecting the level of gastric secretions or which inhibit the release of growth hormones without affecting the level of gastric secretions, insulin and glucagon or the compounds may inhibit the release of gastric acid secretions.

The inventive compounds, furthermore, have a longer duration of activity than somatostatin.

A further object of the present invention, accordingly, are pharmaceutical compositions which are characterized in that they comprise thereapeutically effective amount of the inventive cyclic hexapeptides of formula I or II or a non-toxic acid addition salt of the cyclic hexapeptides of formula I or II.

Depending on the specific compound of formula I or II contained in the inventive pharmaceutical preparations they can be used for the treatment of acromegaly, diabetes, diabetic retinopathy or peptic ulcers. Among said pharmaceutical preparations there are to be found such which are particularly effective when administered to Type I (insulin dependent) diabetics. The active ingredients of said pharmaceutical compositions have the property of preventing large increases in blood sugar which normally occur in such patients following the ingestion of a meal.

In the compounds of formulae I and II the term "lower alkyl" means a straight chain or branched chain alkyl group which has from 1—5 carbon atoms. Examples of such alkyl groups are methyl, ethyl, propyl, iso-ropyl, butyl, sec-butyl or pentyl.

The term "loweralkoxy' is intended to include those alkoxy groups of from 1—5 carbon atoms, in either a straight or branched chain. Examples of such alkoxy groups are methoxy, ethoxy, propoxy, isopropoxy, butoxy, *tert*-butoxy, pentoxy and the like.

The term "halogen" or "halo" is intended to include fluorine, chlorine, bromine and iodine.

The term "5- or 6-membered heterocyclic ring" is intended to include those 5- and 6-membered heterocycles with 1- or 2-heteroatoms selected from oxygen, nitrogen and sulfur. Exemplary of such heterocycles is imidazole, furan, thiazole, pyrazole, pyridine and the like.

In the instant compounds there are several asymmetric centers which will lead to the existence of optical isomers for such compounds. In the instant invention, for each of the asymmetric centers of the various amino acids which make up the instant cyclic hexapeptides, both the D and L configurations are intended to be encompassed.

It will be appreciated by those skilled in the art that when $R_1$ and $R_2$ are benzyl, $R_3$ is indolylmethyl, Y is methylene, and $R_4$ is 1-hydroxyethyl and the 7, 8, 9, 10 and 11 amino acids of somatostatin (-Phe-Trp-Lys-Thr-Phe-) are represented and the secondary amino acid, represented by N-methyl alanine when $R_5$ is methyl, in structure I, and by proline when X is methylene in structure II, has taken the place of the remainder of the somatostatin amino acids. Thus, using the above definitions of the substituent groups, the following representative cyclic hexapeptide analog of somatostatin is formed in structure I;

```
N-Me-Ala-Phe-Trp
      |       |
      Phe-Thr-t-4-AChxAla or AmPhe
```

The preferred embodiments of the cyclic hexapeptides of this invention are realized in the foregoing structural formula I wherein:

$R_1$ and $R_2$ are as defined above;
$R_3$ is 3-indolylmethyl or substituted indolymethyl wherein the substituent is methoxy or fluoro;
$R_4$ is methyl, ethyl, hydroxy methyl or hydroxy ethyl;
$R_5$ is methyl; and
$R_6$ and $R_7$ are hydrogen.

Further preferred embodiments are realized when:
$R_1$ and $R_2$ are as defined above;
$R_3$ is 3-indolymethyl;
$R_4$ is hydroxy ethyl; and
$R_5$ is methyl, ethyl, or benzyl.

The preferred $R_1$ and $R_2$ groups are loweralkyl, benzyl or substituted benzyl where the substituent is loweralkyl, halogen, hydroxy, amino, nitro or alkoxy.

Included within these preferred compounds are:
Cyclo-(N-Me-Ala-Tyr-D-Trp-t-4-AChxAla-Thr-Phe)
Cyclo-(N-Me-Ala-Phe-D-Trp-t-4-AChxAla-Thr-Phe)
Cyclo-(N-Me-Ala-Phe-L-Trp-t-4-AChxAla-Thr-Phe)
Cyclo-(N-Me-Ala-Tyr-D-Trp-t-4-AChxAla-Val-Phe)
Cyclo-(N-Me-Ala-Tyr-L-Trp-t-4-AChxAla-Val-Phe)
Cyclo-(N-Me-Ala-His-D-Trp-t-4-AChxAla-Ser-Phe)
Cyclo-(N-Me-Ala-His-L-Trp-t-4-AChxAla-Ser-Phe)
Cyclo-(N-Me-Phe-Phe-D-Trp-t-4-AChxAla-Thr-Ala)
Cyclo-(N-Me-Phe-Phe-L-Trp-t-4-AChxAla-Thr-Ala)
Cyclo-(N-Me-Phe-Tyr-D-Trp-t-4-AChxAla-Val-Ala)
Cyclo-(N-Me-Phe-Tyr-L-Trp-t-4-AChxAla-Val-Ala)
Cyclo-(N-Me-Abu-Tyr-D-Trp-t-4-AChxAla-Val-Phe)
Cyclo-(N-Me-Abu-His-D-Trp-t-4-AChxAla-Ser-Phe)
Cyclo-(N-Me-Ala-Phe-D-5-F-Trp-t-4-AChxAla-Thr-Phe)
Cyclo-(N-Me-Ala-Phe-L-5-F-Trp-t-4-AChxAla-Thr-Phe)
Cyclo-(N-Me-Ala-Phe-D-Trp-t-4-AChxAla-Ser-Phe)

Cyclo-(N-Me-Ala-Tyr-D-Trp-AmPhe-Val-Phe)
Cyclo-(N-Me-Abu-Phe-D-Trp-AmPhe-Thr-Phe)
Cyclo-(N-Me-Ala-Phe-L-Trp-AmPhe-Thr-Phe)
Cyclo-(N-Me-Phe-Phe-D-Trp-AmPhe-Thr-Ala)
Cyclo-(N-Me-Phe-Phe-L-Trp-AmPhe-Thr-Ala)
Cyclo-(N-Me-Ala-Phe-D-5-F-Trp-AmPhe-Thr-Phe)
Cyclo-(N-Me-Ala-Phe-L-5-F-Trp-AmPhe-Thr-Phe)
Cyclo-(N-Me-Ala-Phe-D-Trp-AmPhe-Ser-Phe)

Using the above definitions of the substituent groups, the following representative cyclic hexapeptide analog of somatostatin is formed in structure II:

```
Pro-Phe-Trp
|       |
Phe-Thr-t-4-AChxAla or AmPhe
```

The preferred embodiments of the cyclic hexapeptides of this invention are realized in the foregoing structural formula II where:

X is $(CH_2)_n$ and n is 1 or 2;
$R_1$ and $R_2$ are as defined above;
$R_3$ is 3-indolylmethyl or substituted indolylmethyl wherein the substituent is methoxy or fluoro; and
$R_4$ is methyl, ethyl, hydroxy methyl or hydroxy ethyl.

Further preferred embodiments are realized when:

X is S or S-methyl;
$R_1$ and $R_2$ are as defined above;
$R_3$ is 3-indolylmethyl; and
$R_4$ is hydroxy ethyl.

The preferred $R_1$ and $R_2$ groups are loweralkyl, benzyl or substituted benzyl where the substituent is loweralkyl, halogen, hydroxy, amino, nitro or alkoxy.

Included within these preferred compounds are:

Cyclo-(Pro-Tyr-D-Trp-t-4-AChxAla-Thr-Phe)
Cyclo-(Pro-Phe-D-Trp-t-4-AChxAla-Thr-Phe)
Cyclo-(Pro-Phe-L-Trp-t-4-AChxAla-Thr-Phe)
Cyclo-(Pro-His-D-Trp-t-4-AChxAla-Ser-Phe)
Cyclo-(Pro-Phe-D-5-F-Trp-t-4-AChxAla-Thr-Phe)
Cyclo-(Pro-Phe-L-5-F-Trp-t-4-AChxAla-Thr-Phe)
Cyclo-(Pro-Phe-D-Trp-t-4-AChxAla-Ser-Phe)
Cyclo-(Pro-Tyr-D-Trp-AmPhe-Val-Phe)
Cyclo-(Pro-Phe-D-Trp-AmPhe-Thr-Phe)
Cyclo-(Pro-Phe-L-Trp-AmPhe-Thr-Phe)
Cyclo-(Pro-His-D-Trp-AmPhe-Ser-Phe)
Cyclo-(Pro-Phe-D-5-F-Trp-AmPhe-Thr-Phe)
Cyclo-(Pro-Phe-L-5-F-Trp-AmPhe-Thr-Phe)
Cyclo-(Pro-Phe-D-Trp-AmPhe-Ser-Phe)

In the instant application several abbreviated designations are used for the amino acid components, certain preferred protecting groups, reagents and solvents. The meanings of such abbreviated designations are given in Table I.

TABLE I

| Abbreviated Designation | Amino Acid |
|---|---|
| Lys | L-lysine |
| Phe | L-phenylalanine |
| Trp | L-tryptophan |
| D-Trp | D-tryptophan |
| Thr | L-threonine |
| Aha | 7-aminoheptanoic acid |
| Tyr | L-tyrosine |
| Val | L-valine |
| Abu | L-α-aminobutyric acid |
| Ser | L-serine |
| Asn | L-asparagine |
| Pro | L-proline |
| Asu | D- or L-aminosuberic acid |
| Cys | L-cysteine |
| AChxAla | amino cyclohexylalanine |
| AmPhe | aminomethyl phenylalanine |
| AChxGly | aminocyclohexylglycine |

| Abbreviated Designation | Protecting Groups |
|---|---|
| INOC | isonicotinyloxycarbonyl |
| BOC | tert-butyloxycarbonyl |
| OMe | methyl ester |
| Bu | tert-butyl |
| CBZ | benzyloxycarbonyl |
| Bzl | benzyl |
| 2-Cl-CBZ | 2-chlorobenzyloxycarbonyl |
| Acm | acetamidomethyl |
| Me | methyl |

| Abbreviated Designation | Activating Groups |
|---|---|
| ONp | p-nitrophenyl ester |
| HSE | N-hydroxysuccinimide ester |
| HBT | 1-hydroxybenzotriazole |

| Abbreviated Designation | Condensing Agents |
|---|---|
| DCCI | dicyclohexylcarbodiimide |

| Abbreviated Designation | Reagents |
|---|---|
| TFA | trifluoroacetic acid |
| TEA | triethylamine |
| DIPEA | diisopropylethylamine |

| Abbreviated Designation | Solvents |
|---|---|
| EPAW | ethyl acetate-pyridine-acetic acid-water |
| BAW | butanol-acetic acid-water |
| CMW | chloroform-methanol-water |
| DMF | dimethylformamide |
| THF | tetrahydrofuran |

In accordance with the present invention, the novel cyclic hexapeptide somatostatin analogs are prepared by cyclizing corresponding linear peptides. The linear peptides are prepared by using the solid phase sequential synthesis technique. Accordingly, the process for preparing the cyclic hexapeptide somatostatin analogs of the present invention comprises a) preparing a corresponding blocked linear peptide attached to a solid phase resin; b) selectively deblocking the N-terminal amine group; c) removing the linear peptide from the resin; d) treating the linear peptide with a cyclizing agent to obtain the cyclic hexapeptide through the formation of an amide bond; e) removing any side chain blocking groups.

When the linear peptide is prepared on the resin, it is generally not critical which amino acid is selected to be at the C-terminal position provided only that the sequence of amino acids in the linear peptide corresponds to that in the desired somatostatin analog. Once a linear peptide has been cyclized one can no longer determine which amino acid was at the C-terminus of the linear peptide.

While generally the selection of the first amino acid to start the chain is not critical, since the linear peptide will be cyclized, there may be other factors which may prefer one starting amino acid over another. For example, D-Trp can react with t-butyl carbonium ions which are formed when BOC groups are removed. Thus, selection of a reaction sequence which places D-Trp at the N-terminal end of the linear peptide will cause D-Trp to be added last, and thus it will have the least exposure to t-butyl carbonium ions. This type of selection may not always be possible, such as where there are two indole containing moieties in the peptide. However, such reaction sensitivities should be considered when planning a peptide reaction sequence.

The synthesis of the linear peptides by the solid phase technique is conducted in a stepwise manner on chloromethylated resin. The resin is composed of fine beads (20—70 microns in diameter) of a synthetic resin prepared by copolymerization of styrene with 1—2 percent divinylbenzene. The benzene rings in the resin are chloromethylated in a Friedel-Crafts reaction with chloromethyl methyl ether and stannic chloride. The Friedel-Crafts reaction is continued until the reaction contains 0.5 to 5 mmoles of chlorine per gram of resin.

The amino acid selected to be the C-terminal amino acid of the linear peptide is converted to its amino protected derivative. The carboxyl group of the selected C-terminal amino acid is bound covalently to the insoluble polymeric resin support, as for example, as the carboxylic ester of the resin-bonded benzyl chloride present in chloromethyl-substituted polystyrene-divinylbenzene resin. After the amino protecting group is removed, the amino protected derivative of the next amino acid in the sequence is added along with a coupling agent, such as dicyclohexylcarbodiimide. The amino acid reactant may be employed in the form of a carboxyl-activated amino acid such as the ONp ester, an amino acid azide, and the like. Deprotection and addition of successive amino acids is performed until the desired linear peptide is formed.

The selection of protecting groups is, in part, dictated by particular coupling conditions, in part by the amino acid and peptide components involved in the reaction.

Amino-protecting groups ordinarily employed include those which are well known in the art, for example, urethane protecting substituents such as benzyloxycarbonyl (carbobenzoxy), p̄-methoxy-carbobenzoxy, p-nitrocarbobenzoxy, t-butyloxycarbonyl, and the like. It is preferred to utilize t-butyloxycarbonyl (BOC) for protecting the α-amino group in the amino acids undergoing reaction at the carboxyl end of said amino acid. The BOC protecting group is readily removed following such coupling reaction and prior to the subsequent step by the relatively mild action of acids (i.e., trifluoroacetic acid, or hydrogen chloride in ethyl acetate).

The OH group of Thr and Ser can be protected by the Bzl group and the ε-amino group of AChxAla and AmPhe can be protected by the INOC group, the benzyloxycarbonyl group (CBZ) or the 2-chlorobenzyloxy-carbonyl (2-Cl-CBZ) group. In the case of AchxAla and AmPhe, it is preferred to protect the ε-amino group with CBZ group as this group is removed simultaneously with the Bzl groups by treatment with HF after the linear peptide as been cyclized. The INOC group is not removed by HF and requires an additional treatment with Zn. Neither group is affected by TFA, used for removing BOC protecting groups. After the linear peptide is cyclized, the protective groups, such as CBZ and Bzl, are removed by treatment with HF.

After the linear peptide has been formed on the solid phase resin, it may be removed from the resin by a variety of methods which are well known in the art. For example, the peptide may be cleaved from the resin with hydrazine and thus directly form the peptide hydrazide which may be subsequently cyclized via the azide to the desired cyclic peptide. The hydrazide is converted to the corresponding azide by reaction with a reagent which furnishes nitrous acid *in situ*. Suitable reagents for this purpose include a lower alkyl nitrite (e.g., n-butyl nitrite, isoamyl nitrite) or an alkyl metal nitrite salt (e.g., sodium nitrite, potassium nitrite) in the presence of a strong acid such as hydrochloric, phosphoric, etc. This reaction is carried out in the presence of either water and/or a non-aqueous solvent such as dimethylformamide, tetrahydrofuran, dioxane, chloroform, methylene chloride, etc., at a temperature between about −40°C and +20°C. Alternatively, the peptide may be removed from the resin by treatment with a lower alcohol such as methanol in the presence of an organic base such as triethylamine, thus resulting in the formation of the corresponding lower alcohol ester of the linear peptide. The resulting ester may be converted to the hydrazide which may then be cyclized, via the azide, to the desired cyclic peptide. The preferred method for cleaving the peptide from the resin in the present invention is the use of hydrazine.

As reference Table II will show, one preferred overall procedure for preparing the desired cyclic

peptides of the present invention involves the stepwise synthesis of the linear peptide on a solid phase resin. More specifically, in the process for preparing:

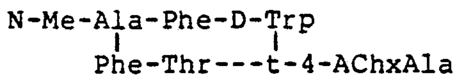

or

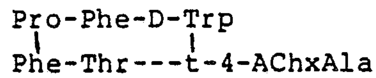

the carboxyl end of the N-blocked amino acid phenylalanine is bound covalently to an insoluble polymeric resin support as the carboxylic acid ester of the resin-bonded benzyl chloride. The amino group of Phe is protected by the BOC group. After the attachment of the Phe is completed on the resin, the protecting group BOC is removed by treatment with TFA in $CH_2Cl_2$. The subsequent amino acids are attached, in the form of BOC-amino acid, using DCCI as the condensing agent or an active ester such as ONp. After the desired linear peptide has been prepared, the N-terminal amino group is selectively deblocked and the peptide is removed from the resin by treatment with hydrazine. The resulting linear peptide hydrazide with the N-terminal amino group deblocked having the amino acid sequence:

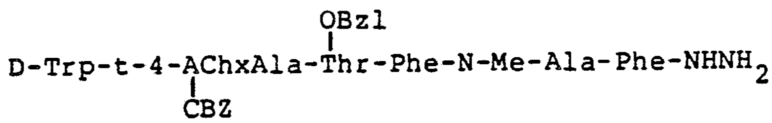

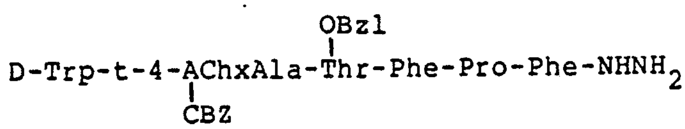

is treated with isoamyl nitrite in acid pH to form the corresponding azide. The azide solution is diluted with solvent and neutralized with an organic base. The linear peptide cyclizes to form:

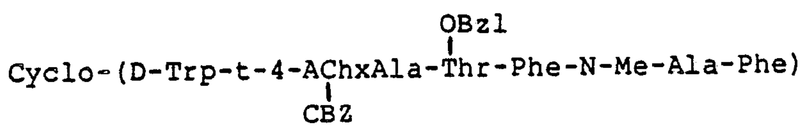

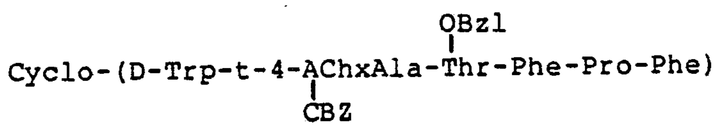

During the cyclization the "pH" is checked and maintained at neutral by the addition of organic base. The "pH" in organic solvent is determined by the application of an aliquot of the solution to moisten narrow range pH paper.

After the linear peptide is cyclized, the protective groups, —CBZ and OBzl, are removed by treatment with HF in the presence of anisole. The crude cyclic peptide obtained is purified chromatographically, preferably with column chromatography on silica gel. The eluant is generally an organic solvent or mixtures thereof which is selected by analyzing aliquots of the material using thin layer chromatography.

TABLE II

The reaction scheme for the preparation of two of the cyclic hexapeptides of this invention is outlined in the following series of reactions:

Reaction scheme for preparing:

N-Me-Ala-Phe-D-Trp
Phe-Thr---t-4-AChxAla

Cl-$CH_2$Ø-resin

↓ BOC-Phe

BOC-Phe-O-$CH_2$Ø-resin

↓ Solid Phase Method
1) BOC-N-Me-Ala
2) BOC-Phe
3) BOC-(OBZl)Thr
4) BOC-(CBZ)-t-4-AChxAla
5) BOC-D-Trp

BOC-D-Trp-CBZ-t-4-AChxAla-(OBZl)Thr-Phe-N-Me-Ala-Phe-O$CH_2$Ø-resin

↓ TFA

D-Trp-CBZ-t-4-AChxAla-(OBZl)Thr-Phe-N-Me-Ala-Phe-O-$CH_2$O-resin

↓ $NH_2NH_2$

D-Trp-CBZ-t-4-AChxAla-(OBZl)Thr-Phe-N-Me-Ala-Phe-$NHNH_2$

↓ isoamyl nitrite, $H^+$, DMF

D-Trp-CBZ-t-4-AChxAla-(OBZl)Thr-Phe-N-Me-Ala-Phe-$N_3$

↓ DMF/triethylamine

Cyclo(D-Trp-CBZ-t-4-AChxAla-(OBZl)Thr-Phe-N-Me-Ala-Phe)

↓ HF/Anisole

Cyclo-(D-Trp-t-4-AChxAla-Thr-Phe-N-Me-Ala-Phe)

Reaction scheme for preparing:

```
Pro-Phe-D-Trp
 |         |
Phe-Thr---t-4-AChxAla
```

Cl-$CH_2$Ø-resin

↓ BOC-Phe

BOC-Phe-O-$CH_2$Ø-resin

↓ Solid Phase Method
1) BOC-Pro
2) BOC-Phe
3) BOC-(OBZl)Thr
4) BOC-(CBZ)-t-4-AChxAla
5) BOC-D-Trp

BOC-D-Trp-CBZ-t-4-AChxAla-(OBZl)Thr-Phe-Pro-Phe-O$CH_2$Ø-resin

↓ TFA

D-Trp-CBZ-t-4-AChxAla-(OBZl)Thr-Phe-Pro-Phe-O-$CH_2$O-resin

↓ $NH_2NH_2$

D-Trp-CBZ-t-4-AChxAla-(OBZl)Thr-Phe-Pro-Phe-$NHNH_2$

↓ isoamyl nitrite, $H^+$, DMF

D-Trp-CBZ-t-4-AChxAla-(OBZl)Thr-Phe-Pro-Phe-$N_3$

↓ DMF/triethylamine

Cyclo(D-Trp-CBZ-t-4-AChxAla-(OBZl)Thr-Phe-Pro-Phe)

↓ HF/Anisole

Cyclo-(D-Trp-t-4-AChxAla-Thr-Phe-Pro-Phe)

The following Examples are given to illustrate the methods used to carry out the present invention. It is to be understood that these Examples are given for purposes of illustration and not limitation.

Example 1

Preparation of D-Trp-(CBZ)-t-4-AChxAla-(OBZl)Thr-Phe-Pro-Phe-$OCH_2$Ø-resin

Chloromethyl resin (2% cross-linked Merrifield resin), 862.0 g (2.37 moles), having 2.75 meq. chlorine/g, and 607.0 g (2.37 moles, 1 equivalent) of BOC-Phe were added to 4320 ml of peroxide-free tetrahydrofuran. The mixture was stirred in an oil bath at 80°C bath temperature for 45 minutes. Triethylamine, 310.0 ml, was added and the reaction mixture stirred at 80°C bath temperature for 70 hours, cooled to 25°C and transferred to a stirred solid phase reaction column with 2000 ml of tetrahydrofuran. After removal of the solvent, the resin was washed using the stirred column with:

3 × 2000 ml of tetrahydrofuran
4 × 5170 ml of ethanol
1 × 5170 ml of acetic acid
3 × 5170 ml of water
3 × 5170 ml of methanol
3 × 5170 ml of chloroform

The BOC-Phe-O—$CH_2$O-resin was dried *in vacuo* at 25°C for 16 hours, giving 1203 g of BOC-Phe-O—$CH_2$O-resin containing 1.2 mmole of phenylalanine/g of resin.

BOC-Phe-O—$CH_2$O-resin (2.13 g; 2.0 mmole) was carried through the procedures in Tables III and IV using 2 deblockings (2 minutes and 25 minutes) with 25% TFA in methylene chloride and 2.5 equivalents of BOC-amino acid in the required sequence until the desired BOC-hexapeptide-O—$CH_2$O-resin was obtained.

DCCI was used as the sole coupling agent in every step.

The coupling of each amino acid proceeded smoothly. Best yields were obtained when the coupling was repeated in each step. When the coupling was repeated, the initial two chloroform washes, the deblocking step and the succeeding three chloroform washes were all omitted and replaced by a single chloroform wash.

The coupling reactions were carried out in methylene chloride, freshly degassed DMF or a mixture of these two solvents. The N-terminal amino group was blocked with a BOC group in each case; the hydroxy group of Thr was blocked with Bzl and the -amino group of t-4-AChxAla with CBZ.

When the desired BOC-hexapeptide-O—$CH_2$O-resin was obtained, the N-terminal BOC group was removed by the terminal deblocking procedure.

TABLE III

| | | | | | | | | |
|---|---|---|---|---|---|---|---|---|
| Solvent or reagent (number of treatments or washes) | $CHCl_3$ (2) | 25% TFA in $CH_2Cl_2$ (2) | $CHCl_3$(3) | $NEt_3$— $CH_2Cl_2$ (1:9) (2) | $CHCl_3$(3) $CH_2Cl_2$(3) | BOC AA in $CH_2Cl_2$ DMF or a mixture of both | 0.5M DCCI in $CH_2Cl_2$ | DMF(1) MeOH(1) DMF(1) MeOH(1) $CHCl_3$(2) |
| Vol. in ml. | 40 | 20 | 40 | 40 | 40 | 25 | 10 | 40 |
| Time in min. | 5 | 2 and 25 | 2 | 5 and 5 | 2 | 5 | 5<br>coupling 30 | 2 |

TABLE IV

| | Protected Amino Acid | Solvent Ml |
|---|---|---|
| I | | |
| | BOC-N-Me-Ala (1.08 g) | 20 ml $CH_2Cl_2$ |
| | Recouple | |
| | BOC-Phe (1.32 g) | 20 ml $CH_2Cl_2$ |
| | Recouple | |
| | BOC(OBzl)Thr (1.55 g) | 20 ml $CH_2Cl_2$ |
| | Recouple | |
| | BOC-[(CBZ)-t-4-AChxAla] (1.5 g) | 20 ml $CH_2Cl_2$ |
| | BOC-D-Trp (1.52 g) | 15 ml $CH_2Cl_2$, 5 ml DMF |
| | Recouple | |
| II | | |
| | BOC-Pro (1.80 g) | 25 ml $CH_2Cl_2$ |
| | Recouple | |
| | BOC-Phe (1.32 g) | 25 ml $CH_2Cl_2$ |
| | Recouple | |
| | BOC(OBzl)Thr (1.55 g) | 25 ml $CH_2Cl_2$ |
| | Recouple | |
| | BOC-[(CBZ)-t-4-AChxAla] (1.5 g) | 25 ml $CH_2Cl_2$ |
| | BOC-D-Trp (1.52 g) | 20 ml $CH_2Cl_2$, 5 ml DMF |
| | Recouple | |

After the procedures of Tables III and IV were completed, the blocked hexapeptide-O—$CH_2$O-resin is dried overnight and weights 3.7 g.

Example 2

Preparation of D-Trp-(CBZ)-t-4-AChxAla-(OBZl)Thr-Phe-N-Me-Ala-Phe-$NHNH_2$

The resin from Example 1 is combined with 30 ml of a 2:1 mixture of dimethylacetamide and hydrazine and stirred at room temperature for 1 hour. The insoluble resin is removed by filtration and the solution is evaporated to remove the dimethylacetamide. The residue is placed under high vacuum overnight to remove all volatile materials. A foam resulted from dissolving the residue in methanol, filtering and evaporation to dryness, weighing 2.19 g.

Example 3

Preparation of D-Trp-(CBZ)-t-4-AChxAla-(OBZl)Thr-Phe-N-Me-Ala-Phe-$N_3$

The foam from Example 2 is combined with 15 ml of degassed dimethylformamide under a blanket of nitrogen and cooled to −10°C, and 5 equivalents of 4.52M hydrogen chloride in tetrahydrofuran (2.2 ml) is added. The solution is cooled to −25°C and 5 ml of a 1:19 mixture of isoamyl nitrite in dimethylformamide is added in portions until a positive starch/KI test is obtained. The completion of the reaction is followed by thin layer chromatography and the disappearance of the hydrazide starting material.

Example 4

Preparation of Cyclo(D-Trp-(CBZ)-t-4-AChxAla-(OBZl)Thr-Phe-N-Me-Ala-Phe)

The azide compound of Example 3 is added to 600 ml of degassed dimethylformamide, precooled to −25°, the pH adjusted to 8, with triethylamine, and the reaction mixture placed in the freezer overnight. The pH is readjusted to 8 if necessary after about 14 hours and the mixture stored for 16 hours at −20°C and 16

hours at 5°C. Thin layer chromatography indicates that the reaction is completed. The mixture is concentrated to dryness, dissolved in 150 ml of a 3:1 dimethylformamide/water mixture and treated with a mixed bed anion-cation exchange resin for 1 hour. The mixture is filtered and concentrated to dryness *in vacuo* to an oil, and the residue is triturated with methanol, affording 1.91 g of a white solid.

Example 5

Preparation of Cyclo-(D-Trp-t-4-AChxAla-Thr-Phe-N-Me-Ala-Phe)

1.91 g (1.8 mmoles) of the protected cyclic hexapeptide of Example 4 is combined in a teflon lined chamber with 2 ml of anisole. The chamber is then evacuated and filled with liquid hydrogen fluoride at the temperature of the dry ice-acetone bath. The temperature is raised to 0°C and stirring continued for 1 hour. The hydrogen fluoride is allowed to evaporate and the residue placed *in vacuo* until a slurry is formed. The slurry is triturated with ethyl acetate and filtered affording 1.29 g of a fine powder. The powder is placed on 300 g of silica gel and eluted with a 10:5:1:3 mixture of EPAW, eluting with 9 ml fractions. Fractions 55—56 are combined and concentrated. The product is precipitated from a mixture of chloroform, ethylacetate and hexane affording 679 mg.

Example 6

Preparation of D-Trp-(CBZ)-t-AChxAla-(OBZl)-Thr-Phe-Pro-Phe-$NHNH_2$

The resin from Example 1 is combined with 30 ml of a 2:1 mixture of dimethylacetamide and hydrazine and stirred at room temperature for 1 hour. The insoluble resin is removed by filtration and the solution is evaporated to remove the dimethylacetamide. The residue is placed under high vacuum overnight to remove all volatile materials. A foam resulted from dissolving the residue in methanol, filtering and evaporating to dryness, weighing 1.84 g.

Example 7

Preparation of D-Trp-(CBZ)-t-AChxAla-(OBZl)-Thr-Phe-Pro-Phe-$N_3$

The foam from Example 2 is combined with 15 ml of degassed dimethylformamide under a blanket of nitrogen and cooled to −10°C, and 5 equivalents of 5.8 M hydrogen chloride in tetrahydrofuran (17 ml) is added. The solution is cooled to −25°C and 5 ml of a 1:19 mixture of isoamyl nitrite in dimethylformamide is added. The completion of the reaction is followed by thin layer chromatography and the disappearance of the hydrazide starting material.

Example 8

Preparation of Cyclo-(D-Trp-(CBZ)-t-4-AChxAla-(OBZl)-Thr-Phe-Pro-Phe)

The azide compound of Example 3 is added to 600 ml of degassed dimethylformamide, precooled to −25°C, the pH adjusted to 8, and the reaction mixture placed in the freezer overnight. The pH is readjusted to 8 if necessary after about 14 hours and the mixture stored for 16 hours at −20°C and 16 hours at 5°C. Thin layer chromatography indicates that the reaction is completed. The mixture is concentrated to dryness, dissolved in 150 ml of 3:1 dimethylformamide/water mixture and treated with a mixed bed anion-cation exchange resin for 2 hours. The mixture is filtered and concentrated to dryness *in vacuo*, and the residue is dissolved in methanol, filtered and evaporated to dryness affording a foam.

Example 9

Preparation of Cyclo(D-Trp-t-4-AChxAla-Thr-Phe-Pro-Phe)

2.13 g (2 mmoles) of the protected cyclic hexapeptide of Example 4 is combined in a teflon lined chamber with 2 ml of anisole. The chamber is then evacuated and filled with liquid hydrogen fluoride at the temperature of the dry ice/acetone bath. The temperature is raised to 0°C and stirring continued for 1 hour. The hydrogen fluoride is allowed to evaporate and the residue placed *in vacuo* until a slurry is formed. The slurry is treated with ethyl acetate and filtered affording 1.19 g of a fine powder. 300 mg of this powder is placed on a 100 μm silica gel preparative layer chromatography plate and eluted with a 70:30:5 mixture of chloroform, methanol and concentrated aqueous ammonia, affording 200 mg of product. The remaining crude product is placed on a silica gel column, and eluted with the same solvent. The presence of the product in the eluent is determined by thin layer chromatographic analysis and, after freeze drying, affords about 510 mg of product.

Following the above procedure, and by modifying only the selection and order of amino acids in the process of Example 1, there are prepared other cyclic hexapeptides of this invention.

The instant cyclic hexapeptide analogs of somatostatin are tested and compared with the effects of somatostatin in an *in vitro* test for the inhibition of growth hormone. The test is described as follows:

"Rat pituitaries were isolated according to the procedures of Vale and Grant "In vitro Pituitary Hormone Secretion Assay for Hypophysiotropic Substances" in Methods in Enzymology, Vol. XXXVII, eds. O'Malley, B. W. and Hardman, J. G. (Academic Press, Inc., New York) pp. 5—93 (1975).

After 4 days in culture, the cells were washed and incubated for 4 hours in Dulbecco-modified Eagle's medium in the presence or absence of graded doses of each analog or somatostatin. The medium was then collected for subsequent growth hormone determination by a double antibody radioimmunoassay for rat growth hormone."

Analogs of somatostatin were compared to somatostatin in their ability to decrease the levels of portal vein glucagon and insulin in anesthetized rats. Male Sprague-Dawley rats (Charles River CD) weighing 160—200 g were anesthetized with urethane (150 mg/100 g of body weight; Aldrich). Saline or peptides were administered via the external jugular vein. After 5 minutes, the portal vein was exposed, and blood was collected via syringe containing 3 mg of EDTA and placed in chilled tubes containing 100 µl of Trasylol (FBA Pharmaceuticals) for subsequent hormone analysis. Plasma levels of glucagon were determined by the method of Faloona and Unger, Methods of Hormone Radioimmunoassay, Jaffe und Behrman (Eds), Academic Press, New York, Vol. II, pp. 257—527 (1976), utilizing glucagon antisera 30K obtained from R. Unger (Dallas, TX). Plasma levels of insulin were determined by a modification of the procedure of Herbert *et al., J. Clin. Endocrinol. Metab., 25,* 1375—1384 (1965).

The test results for some of the compounds of this invention are recorded below with the results for somatostatin listed first and given the arbitrary value of 1. The results for the instant compounds are given as multiples or fractions of the effect of somatostatin. The numbers in parentheses are the fiducial limits for the number preceding. The first of the instant compounds listed is the compound prepared in Examples 1—5. The compound is written slightly different, however, to conform to the order of the amino acids found in somatostatin.

Activity of Cyclic hexapeptide Analogs of Somatostatin

| Compound | Growth Hormone Release Inhibition | Insulin Inhibition | Glucagon Inhibition |
|---|---|---|---|
| Somatostatin | 1 | 1 | 1 |
| Cyclo-(Pro-Phe-D-Trp-*t*-AChxAla-Thr-Phe) | 1.3 (0.9, 1.8) | 57.0 (21.9, 133) | 55.8 (14, 394) |
| Cyclo-(Pro-Phe-L-Trp-*t*-AChxAla-Thr-Phe) | 0.34 (0.14, 0.72) | 0.60 (0.07, 2.3) | 0.29 (0.05, 0.82) |
| Cyclo-(Pro-Phe-D-Trp-*cis*-AChxAla-Thr-Phe) | 0.5 (0.3, 0.6) | 1.4 (0.7, 2.7) | 2.2 (1.1, 4.7) |
| Cyclo-(Pro-Phe-D-Trp-*t*-D-AChxGly-Thr-Phe) | 0.11 | 0.07 | 0.07 |
| Cyclo-(N-Me-Ala-Tyr-D-Trp-*t*-AChxAla-Val-Phe) | 148.5 (133, 164) | 47.5 (25.7, 88.2) | 72.2 (18.9, 320) |
| Cyclo-(N-Me-Ala-Tyr-L-Trp-*t*-AChxAla-Val-Phe) | 26.6 (22.2, 31.7) | 23.2 (11.1, 48.2) | 58.5 (12.9, 85.1) |
| Cyclo-(N-Me-Abu-Tyr-D-Trp-*t*-AChxAla-Val-Phe) | 3.3 (2.0—5.6) | 13.8 (3.7, 42.8) | 28.6 (7.4—112.2) |
| Cyclo-(N-Me-Abu-His-D-Trp-*t*-AChxAla-Ser-Phe) | 1.7 (1.1, 2.7) | 93.4 (39.1, 226) | 88.3 (37.8, 180) |
| Cyclo-(Pro-Phe-D-Trp-*p*-AmPh-Thr-Phe) | | 0.5 (0.3, 0.9) | 0.7 (0.3, 1.6) |

The gastric effects of the instant cyclic hexapeptide analogs of somatostatin are determined by the following procedure.

Compounds were tested for their ability to inhibit pentagastrin evoked gastric secretion in the chronic fistula dog. Female beagle dogs with a chronic gastric fistula were given pentagastrin (2.5 μg./kg./hour, i.v. from 60 to 120 min.) and gastric outputs were collected via the fistula cannula. Samples were analyzed at 30 minute intervals for volume (ml.) and titratable acid (mEq/l) (Titration to pH 7 with 0.01N NaOH); total acid output (mEq) was calculated as the production of output volume and acid concentration. Test compounds were infused at a constant rate from 0 to 60 minutes. Data have been expressed as percent change of total acid output relative to a placebo trial in the same animals.

In the following data, the results for somatostatin are given first for comparison purposes.

Effects of Cyclic hexapeptide Analogs of Somatostatin on Gastric Secretion
(Dose 0.8 Eg/ml./min. — infusion 0—60 min.)

| Compound | Gastric Secretion & Inhibition | | | | | | | | Dose |
|---|---|---|---|---|---|---|---|---|---|
| | Vol. | | | | Acid Concentration | | | | |
| | 0—30 | 30—60 | 60—90 | 90—120 | 0—30 | 30—60 | 60—90 | 90—120 | |
| Somatostatin | 85 | 97 | 81 | 37 | 16 | 77 | 61 | 14 | |
| Cyclo(Pro-Phe-D-Trp-t-AChxAla-Thr-Phe) | 80 | 91 | 50 | 0 | 35 | 49 | 14 | 6 | 0.8 |
| Cyclo(Pro-Phe-D-Trp-*cis*AChxAla-Thr-Phe) | 75 | 82 | 20 | 2 | 4 | 19 | 13 | 5 | 0.8 |

**Claims**

1. A compound having the formula:

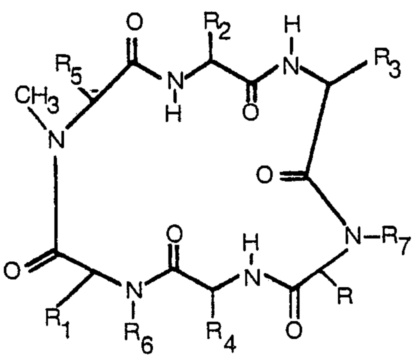

I

or

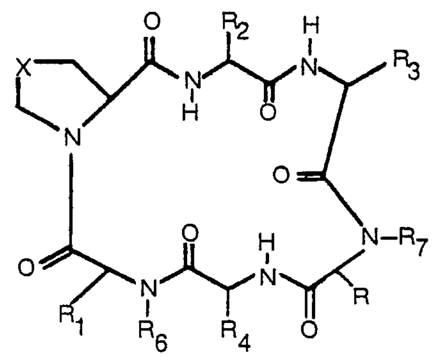

II

wherein

R is

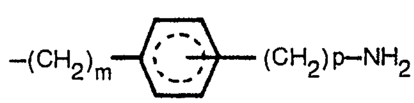

such that the broken line indicates that the ring may be either cyclohexyl or phenyl;

m is 0 or 1; and

p is 0 when the ring is cyclohexyl and 1 when the ring is phenyl;

X is $(CH_2)_n$ wherein n is 0, 1 or 2, or sulfur;

$R_1$ and $R_2$ are independently lower alkyl, benzyl, substituted benzyl wherein the substituent may be one or two of loweralkyl, halogen, hydroxy, amino, nitro or loweralkoxy; and loweralkyl substituted with hydroxy, 3-indolylmethyl, carboxy, amino, guanidino, or a 5- or 6-membered heterocyclic ring;

$R_3$ is 3-indolylmethyl or substituted 3-indolylmethyl wherein the substituent may be loweralkyl, loweralkoxy or halogen;

$R_4$ is loweralkyl, hydroxyloweralkyl, benzyl, carboxyloweralkyl, aminoloweralkyl, 3-indolylmethyl or substituted benzyl or hydroxy-benzyl wherein the substituent may be loweralkyl, loweralkoxy, hydroxy, halogen, amino or nitro, or a 5- or 6-membered heterocyclic ring;

$R_5$ is loweralkyl, benzyl, or substituted benzyl wherein the substituent is loweralkyl, loweralkoxy, hydroxy, halogen, amino or nitro; and

$R_6$ and $R_7$ are independently hydrogen or methyl,

and salts of the compounds of formula I or II.

2. A compound of Claim 1 wherein: X is $(CH_2)_n$ and n is 1; $R_1$ and $R_2$ are as defined in Claim 1; $R_3$ is 3-indolylmethyl or substituted indolylmethyl wherein the substituent is methoxy or fluoro; $R_4$ is methyl, ethyl, hydroxymethyl or hydroxyethyl; and $R_5$ is methyl, $R_6$ and $R_7$ are hydrogen.

3. A compound of Claim 2 wherein: X and Y are methylene; $R_1$ and $R_2$ are as defined in Claim 1, $R_3$ is 3-indolylmethyl; $R_4$ is hydroxyethyl; and $R_5$ is methyl, $R_6$ and $R_7$ are hydrogen.

4. The compound of Claim 2 which is cyclo (Pro-Tyr-D-Trp-t-4-AChxAla-Thr-Phe).

5. The compound of Claim 2 which is cyclo (Pro-Phe-D-Trp-t-4-AChxAla-Thr-Phe).

6. The compound of Claim 2 which is cyclo (N-Me-Ala-Tyr-D-Trp-t-4-AChxAla-Thr-Phe).

7. A process for preparing a cyclic hexapeptide compound of Claim 1 which comprises:

a) preparing a corresponding blocked linear peptide attached to a solid phase resin;

b) selectively deblocking the N-terminal amine group;

c) removing the linear peptide from the resin;

d) treating the linear peptide with a cyclizing agent to form the amide bond of the desired cyclic hexapeptide;

e) removing the side chain protecting groups.

8. The process of Claim 7 wherein step c) comprises treating the linear peptide resin with hydrazine to form the hydrazide.

9. Pharmaceutical composition characterized in that it comprises a therapeutically effective amount of the cyclic hexapeptides of formula I or II according to claim 1 or non-toxic acid addition salts of the cyclic hexapeptides of formula I or II.

10. Pharmaceutical composition according to patent claim 9, characterized in that it, furthermore, comprises a pharmaceutically acceptable liquid or solid carrier.

**Patentansprüche**

1. Verbindung mit der Formel

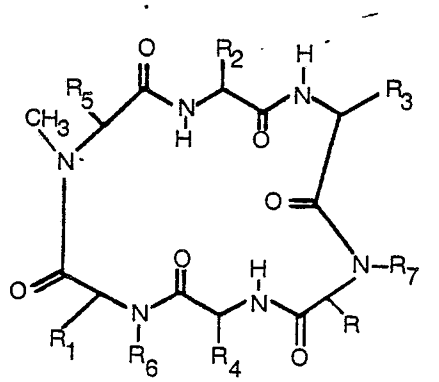

oder

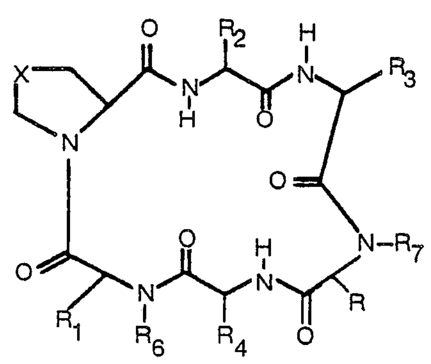

I

II

worin R

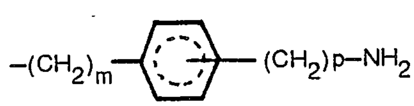

ist, wobei die gestrichtelte Linie anzeigt, daß der Ring entweder Cyclohexyl oder Phenyl sein kann;

m 0 oder 1 ist; und

p 0 ist, wenn der Ring Cyclohexyl ist, und 1, wenn der Ring Phenyl ist;

X $(CH_2)_n$, worin n 0, 1 oder 2 ist, oder Schwefel ist;

$R_1$ und $R_2$ unabhängig Niedrigalkyl, Benzyl, substituiertes Benzyl, worin der Substituent eines oder zwei aus Niedrigalkyl, Halogen, Hydroxy, Amino, Nitro oder Niedrigalkoxy sein kann; und mit Hydroxy, 3-Indolylmethyl, Carboxy, Amino, Guanidino oder einem 5- oder 6-gliedrigem heterocyclischen Ring substituiertes Niedrigalkyl sind;

$R_3$ 3-Indolylmethyl oder substituiertes 3-Indolylmethyl, worin der Substituent Niedrigalkyl, Niedrigalkoxy oder Halogen sein kann, ist;

$R_4$ Niedrigalkyl, Hydroxyniedrigalkyl, Benzyl, Carboxyniedrigalkyl, Aminoniedrigalkyl, 3-Indolylmethyl oder substituiertes Benzyl oder Hydroxybenzyl, worin der Substituent Niedrigalkyl, Niedrigalkoxy, Hydroxy, Halogen, Amino oder Nitro oder ein 5- oder 6-gliedriger heterocyclischer Ring sein kann.

$R_5$ Niedrigalkyl, Benzyl oder substituiertes Benzyl, worin der Substituent Niedrigalkyl, Niedrigalkoxy, Hydroxy, Halogen, Amino oder Nitro ist, ist; und

$R_6$ und $R_7$ unabhängig Wasserstoff oder Methyl sind,

und Salze der Verbindungen der Formel I oder II.

2. Verbindung nach Anspruch 1, worin X $(CH_2)_n$ ist und n 1 ist; $R_1$ und $R_2$ wie in Anspruch 1 definiert sind; $R_3$ 3-Indolylmethyl oder substituiertes Indolylmethyl ist, worin der Substituent Methoxy oder Fluor ist; $R_4$ Methyl, Ethyl, Hydroxymethyl oder Hydroxyethyl ist, und $R_5$ Methyl ist, $R_6$ und $R_7$ Wasserstoff sind.

3. Verbindung nach Anspruch 2, worin X und Y Methylen sind; $R_1$ und $R_2$ wie in Anspruch 1 definiert sind, $R_3$ 3-Indolylmethyl ist; $R_4$ Hydroxyethyl ist; und $R_5$ Methyl ist, $R_6$ und $R_7$ Wasserstoff sind.

4. Verbindung nach Anspruch 2, welche Cyclo(Pro-Tyr-D-Trp-t-4-AChxAla-Thr-Phe) ist.

5. Verbindung nach Anspruch 2, welche Cyclo(Pro-Phe-D-Trp-t-4-AChxAla-Thr-Phe) ist.

6. Verbindung nach Anspruch 2, welche Cyclo(N-Me-Ala-Tyr-D-Trp-t-4-AChxAla-Thr-Phe) ist.

7. Verfahren zur Herstellung einer cyclischen Hexapeptidverbindung nach Anspruch 1 durch

a) Herstellung eines entsprechenden, an ein Harz mit fester Phase gebundenen blockierten linearen Peptids;

b) selektives Entfernen der Blockierungsgruppen der N-terminalen Aminogruppe;

c) Entfernen des linearen Peptids vom Harz;

d) Behandeln des linearen Peptids mit einem Cyclisierungsmittel unter Bildung der Amidbindungg des erwünschten cyclischen Hexapeptids;

e) Entfernen der Seitenkettenschutzgruppen.

8. Verfahren nach Anspruch 7, worin Schritt c) das Behandeln des linearen Peptidharzes mit Hydrazin unter Bildung des Hydrazids umfaßt.

9. Pharmazeutische Zusammensetzung, dadurch gekennzeichnet, daß sie eine therapeutisch wirksame Menge der cyclischen Hexapeptide der Formel I oder II nach Anspruch 1 oder nicht toxische Säureadditionssalze der cyclischen Hexapeptide der Formel I oder II umfaßt.

10. Pharmazeutische Zusammensetzung nach Patentanspruch 9, dadurch gekennzeichnet, daß sie weiterhin einen pharmazeutisch annehmbaren flüssigen oder festen Träger umfaßt.

**Revendications**

1. Composé répondant à la formule

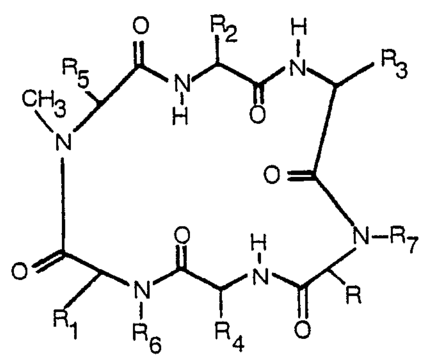

ou

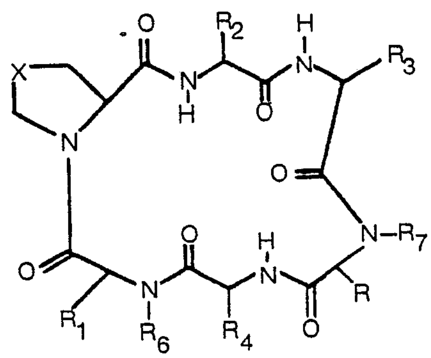

I

II

dans laquelle

R est

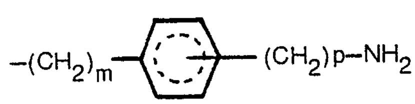

et tel que le trait en tirets indique que le noyau peut être le noyau cyclohexyle ou phényle;

m vaut 0 ou 1; et

p vaut 0 lorsque le noyau est le cyclohexyle et 1 lorsque le noyau est le phényle;

X est $(CH_2)_n$ où n vaut 0, 1 ou 2, ou un atome de soufre;

$R_1$ et $R_2$ sont indépendamment un groupe alkyle inférieur, benzyle, benzyle substitué dans lequel le substituant peut être un ou deux groupes choisis parmi les groupes alkyle inférieur, halogène, hydroxyle, amino, nitro ou alcoxy inférieur; et alkyle inférieur substitué par un groupe hydroxyle, 3-indolyméthyle, carboxyle, amino, guanidino, ou un noyau hétéroxyclique à 5 ou 6 chaînons;

$R_3$ est le groupe 3-indolylméthyle ou un groupe 3-indolylméthyle substitué dans lequel le substituant peut être un groupe alkyle inférieur, alcoxy inférieur ou halogène;

$R_4$ est un groupe alkyle inférieur, hydroxy-alkyle inférieur, benzyle, carboxy-alkyle inférieur, amino-alkyle inférieur, 3-indolyméthyle ou benzyle ou hydroxy-benzyle substitué où le substituant peut être un groupe alkyle inférieur, alcoxy inférieur, hydroxyle, halogène, amino ou nitro, ou un noyau hétérocyclique à 5 ou 6 chaînons;

$R_5$ est un groupe alkyle inférieur, benzyle ou benzyle substitué où le substituant est un groupe alkyle inférieur, alcoxy inférieur, hydroxyle, halogène, amino ou nitro; et

$R_6$ et $R_7$ sont indépendamment un atome d'hydrogène ou le groupe méthyle,

et les sels des composés de formule I ou II.

2. Composé selon la revendication 1, dans lequel: X est $(CH_2)_n$ et n vaut 1; $R_1$ et $R_2$ sont tels que définis dans la revendication 1; $R_3$ est le groupe 3-indolyméthyle ou un groupe indolylméthyle substitué dans lequel le substituant est le groupe méthoxy ou un atome de fluor; $R_4$ est le groupe méthyle, éthyle, hydroxyméthyle ou hydroxyéthyle; et $R_5$ est le groupe méthyle; et $R_6$ et $R_7$ sont un atome d'hydrogène.

3. Composé selon la revendication 2, dans lequel: X et Y sont le radical méthylène; $R_1$ et $R_2$ sont tels que définis dans la revendication 1; $R_3$ est le groupe 3-indolylméthyle; $R_4$ est le groupe hydroxyéthyle; $R_5$ est le groupe méthyle; et $R_6$ et $R_7$ sont un atome d'hydrogène.

4. Composé selon la revendication 2, qui est le cyclo (Pro-Tyr-D-Trp-t-4-AChxAla-Thr-Phe).

5. Composé selon la revendication 2, qui est le cyclo (Pro-Phe-D-Trp-t-4-AChxAla-Thr-Phe).

6. Composé selon la revendication 2, qui est le cyclo (N-Me-Ala-Tyr-D-Trp-t-4-AChxAla-Thr-Phe).

7. Procédé pour la préparation d'un composé hexapeptide cyclique selon la revendication 1, qui comprend:

a) la préparation d'un peptide linéaire bloqué correspondant, fixé à une résine en phase solide;

b) le déblocage sélectif du groupe amine N-terminal;

c) la séparation du peptide linéaire de la résine;

d) le traitement du peptide linéaire avec un agent de cyclisation pour former la liaison amide de l'hexapeptide cyclique souhaité;

e) l'élimination des groupes protecteurs des chaînes latérales.

8. Procédé selon la revendication 7, dans lequel l'étape c) comprend le traitement de la peptide linéaire-résine avec l'hydrazine pour former l'hydrazide.

9. Composition pharmaceutique caractérisée en ce qu'elle comprend une quantité thérapeutiquement efficace des hexapeptides cycliques de formule I ou II selon la revendication 1 ou des sels non toxiques d'addition d'acides des hexapeptides cycliques de formule I ou II.

10. Composition pharmaceutique selon la revendication 9, caractérisée en ce qu'elle comprend en outre un véhicule liquide ou solide, pharmaceutiquement acceptable.